# FASCICULE DE BREVET EUROPEEN

(11) **EP 1 587 561 B1**
(45) Date de publication et mention de la délivrance du brevet: **19.03.2008**
(21) Numéro de dépôt: 04703416.0
(22) Date de dépôt: 20.01.2004
(51) Int. Cl.: A61M 5/148

(54) **DISPOSITIF D INJECTION DE LIQUIDE MEDICAL**
VORRICHTUNG ZUR INJEKTION MEDIZINISCHER FLÜSSIGKEIT
MEDICAL LIQUID INJECTION DEVICE

(30) Priorité: 21.01.2003 FR 0300599
(43) Date de publication de la demande: 26.10.2005
(73) Titulaire: MEDEX S.A., 74940 Annecy Le Vieux (FR)
(72) Inventeur: LACROIX, Jean-Pierre, F-74940 Annecy Le Vieux (FR); LAMY, Didier, F-74330 Poisy (FR)
(74) Mandataire: Gasquet, Denis
(86) Numéro de dépôt international: PCT/FR2004/000109
(87) Numéro de publication internationale: WO 2004/067054

(56) Documents cités:
- EP-A- 0 676 214
- EP-B- 0 343 286
- WO-A-01/03758
- FR-A- 2 619 860
- US-A- 3 199 511
- US-A- 4 023 587
- US-A- 4 425 114
- US-A- 5 230 610
- US-A- 5 450 743

## Description

La présente invention concerne un dispositif d'injection d'un liquide médical destiné au corps humain ou animal. Il est plus particulièrement relatif à un perfectionnement permettant de mettre en pression le liquide médical à injecter quand il est contenu dans une enveloppe souple.

Les récents développements de la médecine ont conduit notamment à mettre au point différents procédés d'analyse et de contrôle de l'état des patients. Parmi ces procédés il existe les analyses faites après injection d'un produit de contraste. C'est par exemple le cas pour les analyses du type angiographie qui consistent à injecter un produit iodé dans les vaisseaux, veines et artères pour opacifier l'ensemble de ceux-ci ainsi que les tissus et organes imprégnés d'opacifiant et ainsi, par radio, déterminer les anomalies éventuelles, comme les thromboses, les embolies, les compressions et autres. Il peut s'agir aussi d'utilisation de produits de contraste pour réaliser un scanner ou en angiographie traditionnelle ou numérisée, l'injection veineuse ou artérielle devant se faire sous forme de bolus à vitesses allant généralement de 0,5 à 35 ml/s. Mais il existe aussi bien d'autres types d'injection sans pour autant que ce soient des injections de liquide de contraste. C'est par exemple le cas des transfusions ou des procédés de nutrition artificielle, notamment par le sang ou par voie digestive (entérale ou parentérale).

On connaît déjà des injecteurs qui comprennent des seringues contenant le liquide à injecter dont la tête d'injection est reliée à une aiguille hypodermique ou intraveineuse ou à un cathéter par l'intermédiaire d'une canalisation qui comprend éventuellement une valve anti-retenue à seuil. La préparation des seringues actuelles est faite par remplissage et débullage à partir de flacons de différentes contenances telles que 60, 100, 200 millilitres, voire même plus, comme, par exemple, 500 millilitres. Devant l'exigence croissante des utilisateurs dans le domaine de l'hygiène et de la non-contamination par les patients des produits utilisés, qui peuvent servir à plusieurs patients, il est donc souhaitable d'éviter au maximum les transvasements de flacon à seringue surtout si les seringues sont appelées à servir pour plusieurs patients. Le but étant d'éviter tout risque de contamination rétrograde. Le développement des maladies contagieuses, notamment du Sida, conduit les médecins à une exigence encore plus importante dans le domaine de l'hygiène et il est donc déterminant et très important de supprimer tout risque de contamination et donc de supprimer au maximum les transvasements de produits initialement nécessaires et ce dans un but principal d'hygiène et de sécurité. Les constructeurs ont donc imaginé des dispositifs dans lesquels le liquide médical est utilisé directement dans un conditionnement constitué par une poche souple munie d'un raccord. Ladite poche remplaçant ainsi la seringue traditionnelle qui est connectée à une canalisation reliée à un conduit d'injection tel qu'un cathéter ou une aiguille hypodermique, intraveineuse ou autre, est placée dans un boîtier comprenant le liquide moteur inerte mis sous pression.

De tels dispositifs sont, par exemple, décrits dans les demandes de brevets européens 0 343 286 et 0 676 214, dans le brevet américain 3 199 511, ainsi que dans la demande de brevet française 2 592 306. Dans tous ces dispositifs il est fait usage d'une poche souple contenant le liquide à injecter, laquelle poche est placée dans une enceinte contenant le liquide moteur qui, mis sous pression, aura pour mission de propulser le liquide contenu dans la poche. Cependant, si de tels dispositifs ont permis des progrès considérables dans le domaine de l'injection, ils présentent néanmoins des inconvénients liés à leur utilisation et à leur mode de construction.

Il est aussi connu par le brevet français publié sous le numéro 2 758 088, un dispositif perfectionné, selon lequel la poche contenant le liquide médical qui est molle et déformable est mise en place dans le liquide moteur d'un boîtier. La poche est placée verticalement, tandis que le liquide moteur enveloppant la totalité de la poche et étant mise en pression pour déformer ladite poche de façon homogène et périphérique, afin de forcer le liquide médical à s'écouler par le conduit de sortie. Selon ce dispositif les moyens de mise en pression du liquide moteur sont constitués par un vérin qui se déplace grâce à une vis mise en rotation par un moteur. Ce type de dispositif bien que très perfectionné, par rapport aux matériels existant, n'est toute fois pas totalement parfait, surtout en ce qui concerne la fiabilité des volumes de liquide injecté, ainsi qu'en ce qui concerne leur poids, leur encombrement, et leur coût.

La présente invention entend donc résoudre les inconvénients ci-dessus mentionnés en proposant une solution nouvelle relative aux moyens de mies en pression du liquide moteur.

Ainsi, le dispositif d'injection de l'invention, destiné à mettre en oeuvre un procédé d'injection de liquide à usage médical du type qui consiste
- à utiliser une poche souple contenant ledit liquide médical à injecter, ladite poche étant étanche et comprenant au moins un conduit de sortie destiné à être connecté à un conduit d'injection tel qu'un cathéter ou une aiguille hypodermique, intraveineuse ou autre,
- à déformer la poche souple par l'intermédiaire d'un liquide moteur inerte contenu dans un réservoir, mis en pression de façon à transmettre cette pression au liquide médical à injecter afin de le forcer à s'écouler dans le conduit de sortie en la plaçant dans l'enceinte interne, d'un boîtier comprenant le liquide moteur inerte, puis en mettant en pression ledit liquide moteur,
est caractérisé en ce que les moyens de mise en pression du liquide moteur sont constitués par une pompe volumétrique à pistons mise en mouvement par un moteur électrique.

Selon une caractéristique complémentaire, les moyens de mise en pression comprennent un premier distributeur relié à la pompe ainsi qu'au réservoir et à l'enceinte interne, ledit distributeur étant commandé par un circuit électronique pour permettre soit le déplacement du liquide du réservoir vers l'enceinte, soit inversement le déplacement du liquide de l'enceinte vers le réservoir.

Selon une autre caractéristique, l'enceinte motrice contenant le liquide moteur est constituée par le corps du boîtier et une enceinte déformable réalisée en matériau souple et déformable élastiquement.

Ajoutons que selon une variante de réalisation, la poche souple comprend deux compartiments comprenant deux liquides différents, et deux conduits de sortie, tandis qu'il est prévu un pince conduits actionné par un bloc de commande de façon à condamner l'un ou l'autre des conduits de sortie.

Notons que le pince conduits est constitué par un étrier mobile comprenant deux pointeaux internes dirigés l'un vers l'autre, tandis qu'une butée centrale d'appui est prévue au centre de l'étrier.

D'autres caractéristiques et avantages de l'invention se dégageront de la description qui va suivre en regard des dessins annexés qui ne sont donnés qu'à titre d'exemples non limitatifs.

La figure 1 est une vue illustrant de façon schématique l'ensemble du dispositif d'injection.

Les figures 2 et 3 sont des vues schématiques illustrant un premier mode de réalisation.

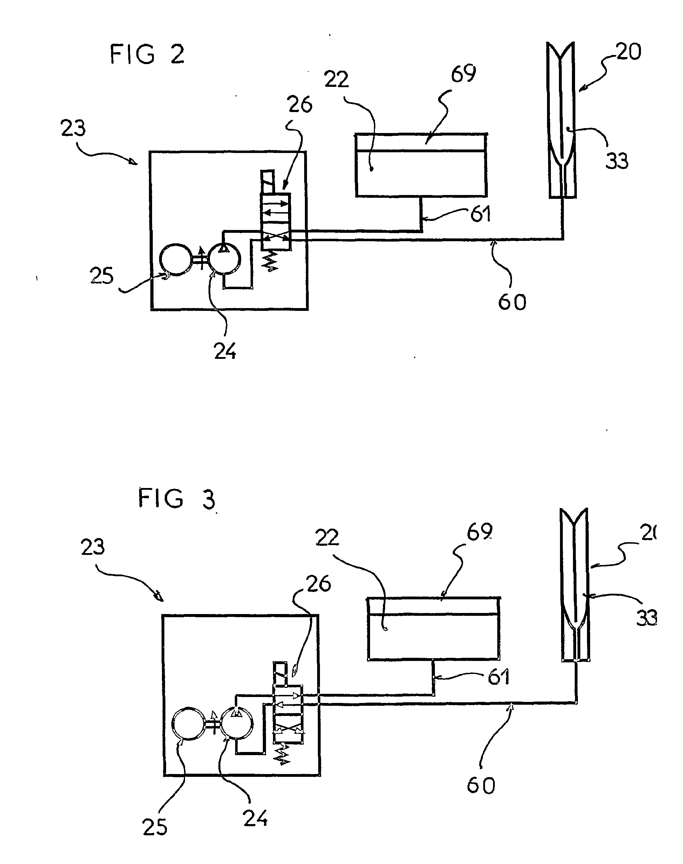

La figure 2 est une vue lors de la mise en pression du liquide moteur.

La figure 3 est une vue lors du retour du liquide moteur vers le réservoir.

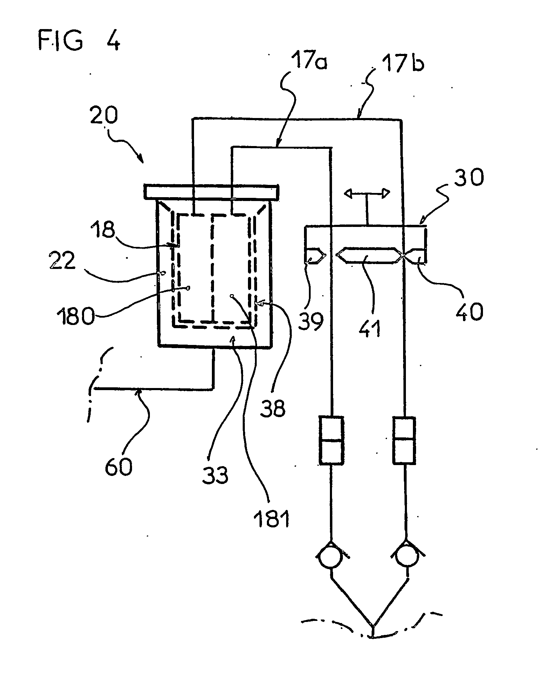

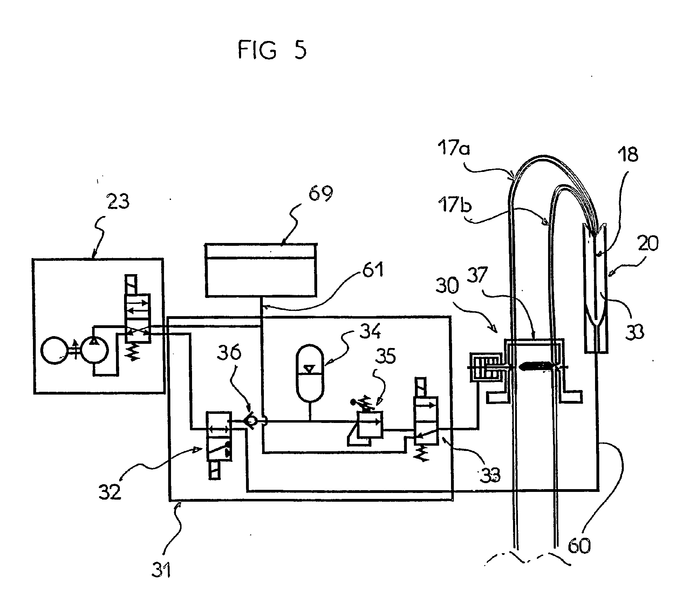

Les figures 4 et 5 sont des vues schématiques illustrant une variante de réalisation.

La figure 4 est une vue représentant le boîtier avec une poche souple comprenant deux compartiments, chacun deux contenant un liquide différent.

La figure 5 est une vue similaire à la figure 2, l'un des conduits de sortie de poche étant condamné, par le pince conduits.

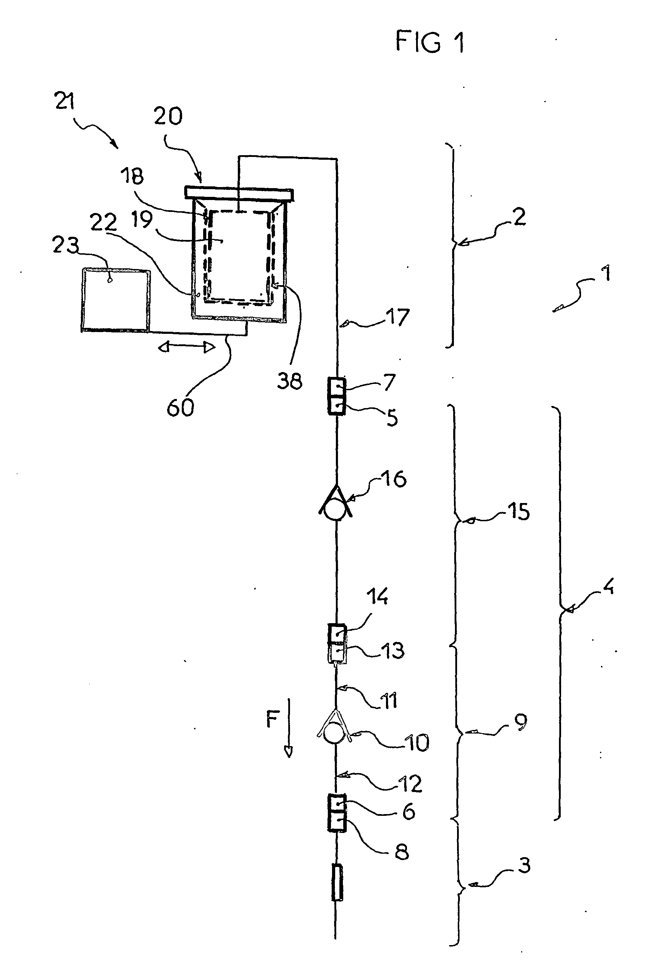

La figure 1 illustre schématiquement l'ensemble du dispositif d'injection selon l'invention qui porte la référence globale (1). Ledit dispositif comprend un injecteur proprement dit ou dispositif d'alimentation (2) relié à un conduit d'injection (3) tel qu'un cathéter, une aiguille hypodermique ou intraveineuse par l'intermédiaire d'une canalisation (4) constituée de plusieurs éléments. Ladite canalisation (4) comprend, à ses extrémités, deux raccords de branchement, un raccord amont (5) et un raccord aval (6). Le raccord amont (5) est destiné à être raccordé à un raccord d'alimentation (7) solidaire de l'injecteur, tandis que le raccord aval (6) est destiné à être raccordé au raccord amont (8) du conduit d'injection (3). Par ailleurs et avantageusement, la canalisation (4) comprend un dispositif de sécurité (9) tel que décrit dans la demande française n° 93 12590 et qui est constitué par un raccord amovible comprenant une valve anti-retour à seuil (10) comprise entre une canalisation amont (11) et une canalisation aval (12). La valve anti-retour à seuil (10) est adaptée pour autoriser le passage du liquide d'amont en aval comme représenté par la flèche "F" lorsqu'une pression déterminée du fluide en écoulement est atteinte, tandis qu'elle condamne le passage du liquide médical en sens inverse, à savoir, d'aval en amont, c'est-à-dire dans le sens contraire de celui illustré par la flèche "F". Notons que la canalisation amont (11) du dispositif de sécurité (9) comprend un raccord amont (13) destiné à être raccordé à un raccord aval (14) d'une portion amont de la canalisation (4) qui comprend par ailleurs une autre valve anti-retour à seuil (16), tandis que la canalisation aval (12) comprend le raccord aval (6) précédemment décrit.

Ajoutons que la canalisation (4) est connectée à l'injecteur (2) par raccordement du raccord amont (5) de ladite canalisation au raccord d'alimentation (7), qui est directement fixé à l'extrémité du conduit de sortie (17) d'une poche souple (18) contenant le liquide médical (19) à injecter. Ladite poche souple (18) est disposée dans un boîtier de mise en pression (20), tandis que la mise en pression de la poche souple est réalisée par un liquide moteur inerte enveloppant ladite poche. Ledit liquide moteur inerte (22) étant contenu dans le boîtier (20), et sa mise en pression étant réalisée par des moyens de mise en pression (23).

Le boîtier (20) est par exemple du type de celui décrit dans le brevet français publié sous le numéro 2758088. En conséquence, il ne sera pas décrit en détails, l'invention portant par ailleurs sur les moyens de mise en pression. On notera seulement ce qui suit :

La poche (18) contenant le liquide médical (19) est molle et déformable et est, selon l'invention, mise en place dans le liquide moteur (22) d'un boîtier (20), ledit liquide enveloppant la totalité de la poche et étant mise en pression pour déformer ladite poche de façon homogène et périphérique, afin de forcer le liquide médical à s'écouler par le conduit de sortie (17). Ajoutons que le boîtier (20) comprend une enceinte déformable (38) destinée à recevoir la poche souple (18). Ainsi, le liquide moteur (22) est disposé dans l'enceinte (33) formée entre le corps du boîtier et l'enceinte déformable, de façon à ce que le liquide moteur (22) ne soit pas en contact direct avec la poche souple (18).

Notons aussi que l'enceinte déformable (38) est réalisée dans un matériau souple et déformable tel qu'en Latex, en Néoprène, en silicone ou en élastomère polydiméthyle. Notons, par ailleurs, que le corps de boîtier (30) peut avantageusement être réalisé en aluminium ou en acier, par exemple, de manière à pouvoir mettre le liquide moteur en pression maximale comprise entre 80 à 100 bars.

Le liquide moteur inerte (22) peut, par exemple, être de l'eau, de la glycérine ou un mélange des deux. De plus, afin de faciliter l'enlèvement des bulles d'air indésirables que pourrait contenir la poche souple (18), le boîtier (20) est orienté de manière à ce que l'orifice d'écoulement de ladite poche reliée à la canalisation débouche vers le haut,, l'ouverture supérieure du corps de boîtier (30) étant située à l'extrémité supérieure dudit corps.

L'enceinte (33) est alimentée en liquide moteur (22) par un conduit d'arrivée (60) connecté aux moyens de mise en pression (23), connecté par ailleurs par une tubulure (61) à un réservoir de liquide moteur (69).

Selon l'invention les moyens de mise en pression du liquide moteur comprennent une pompe (24) mise en mouvement par un moteur électrique (24).

La pompe (24) est une pompe volumétrique à pistons, dont la succession de pistons disposés circulairement sont mis en mouvement par un plateau tournant, permettant de provoquer le déplacement d'un volume constant de liquide moteur quelle que soit la vitesse du plateau, car à chaque tour du plateau correspond un volume déterminé de mise en pression du liquide moteur. Par exemple chaque tour de plateau mettra en pression cinq millilitre de liquide moteur. Ce type de pompe permet d'obtenir un débit régulier et constant de liquide moteur afin d'assurer un débit de produit injecté lui aussi régulier et constant, par une injection rapide qui cesse dans la seconde qui suit l'arrêt de la mise en pression du liquide moteur. Ajoutons que ce type de pompe délivre un volume te un débit constant quelle que soit la pression et ce pour une vitesse de rotation donnée, ce qui permet de transférer dans la poche motrice un volume strictement proportionnel au nombre de tour du moteur et ce quelle que soit la pression. Grâce à l'utilisation d'une telle pompe, le dispositif ne nécessite pas de capteur et d'asservissement particulier.

Ajoutons que la pompe tourne toujours dans le même sens que ce soit pour mettre en pression le liquide moteur dans l'enceinte (33), ou pour vider ladite enceinte, aussi, il est prévu un distributeur (26) pour provoquer les différents déplacements du liquide moteur.

Les figures 2 et 3 représentent un premier mode de réalisation des moyens de mise en pression du liquide moteur, constitués par la pompe (24) et son moteur (25) ainsi que le premier distributeur (26). Sont représentés aussi le boîtier (20) avec son enceinte (33) et le réservoir (69) de liquide moteur (22). Bien entendu le distributeur (26) est commandé par une électronique qui n'a pas été représentée.

A la figure 2, le dispositif est dans une configuration selon laquelle la position du distributeur permet le passage du liquide moteur du réservoir (69) vers l'enceinte (33). Lorsque l'enceinte motrice (33) est comprimée à une pression déterminée, par exemple 2 bars, le distributeur est activé, pour diriger le liquide moteur de l'enceinte motrice (33) vers le réservoir (69), tel que cela est illustré à la figure 3.

### La remise à zéro se fait comme suit :

La pompe (24) est mise en route à une vitesse déterminée, telle que par exemple pour obtenir 20ml/s, puis elle est arrêtée lorsque la poche motrice est comprimée à une pression déterminée, comme par exemple 2 bars. Le compteur est ensuite mis à zéro. Puis le premier distributeur (26) est activé pour diriger le fluide moteur (22) de la poche motrice vers le réservoir (69), et ce pour pomper un volume déterminé et se retrouver dans la position initiale. Le dispositif est ensuite arrêté et prêt pour une injection.

Les figures 4 et 5 représentent une variante de réalisation selon laquelle la poche souple (18') comprend deux compartiments (180, 181) comprenant deux liquides différents, comme par exemple d'une part du gadolinium (181), et d'autre part du sérum physiologique (180). A cet effet la poche souple (18') comprend deux conduits de sortie (17a, 17b) tel que cela est illustré à la figure 4.

Selon cette variante de réalisation et comme précédemment, les moyens de mise en pression du liquide moteur, sont constitués par la pompe (24) et son moteur (25) ainsi que le distributeur (26) commandé par une électronique qui n'a pas été représentée, mais selon cette variante il est prévu un pince conduits (30) commandé par des moyens de commande de pince (31), constituant un deuxième bloc de commande avec les moyens de mise en pression (23). Ledit pince conduits est, par exemple, disposé sur le côté du boîtier (20).

Par ailleurs le deuxième bloc (31) comprend deux distributeurs, un deuxième distributeur (32), et un troisième distributeur (33), un accumulateur (34), ainsi qu'un régulateur de pression (35) et une vanne anti-retour (36).

Le deuxième bloc de commande (31) permet d'actionner le pince conduits (30) façon sélective, pour laisser passer soit le sérum physiologique, soit le gadolinium tel que cela est illustré aux figures 4 et 5.

On notera que la tubulure (61) du réservoir de liquide moteur est reliée, d'une part, au premier distributeur (26) et, d'autre part, au troisième distributeur (33). Par ailleurs, le deuxième distributeur est relié au premier distributeur (26) et au troisième distributeur (33), tandis que le conduit d'arrivée (60) est relié au deuxième distributeur (32), le pince conduits (30) étant relié au troisième distributeur (33).

La mise à zéro du dispositif se fait comme suit

A la mise sous tension, le premier distributeur (32) est actionné pour gonfler l'accumulateur (34), et la pompe est mise en route. Lorsque l'accumulateur est gonflé à une pression déterminée telle que, par exemple, 50 bars (afin de constituer une énergie potentielle), il y a arrêt de la pompe et désactivation du premier distributeur (32). Il est procédé ensuite à la mise en route de la pompe et son arrêt est ensuite provoqué lorsque la poche motrice est comprimée à une pression déterminée comme par exemple à 2 bars. Ensuite on procède à la mise à zéro du compteur avant d'activer le premier distributeur (26) des moyens de mise en pression (23) pour diriger le fluide moteur de la poche motrice vers le réservoir. Une fois que le liquide est restitué au réservoir, le dispositif est prêt pour une injection.

### Le retour à la position initiale après injection procède comme suit:

A la fin de l'injection la pompe (24) s'arrête, le distributeur (26) est activé pour diriger le fluide de l'enceinte motrice (33) vers le réservoir (69). Il y a ensuite désactivation du deuxième distributeur (32) pour procéder au gonflage de l'accumulateur à une pression déterminée, comme par exemple à 50 bars. La pompe (24) est remise en route et s'arrête lorsque l'accumulateur est gonflé à une pression déterminée telle que par exemple 50 bars, pour constituer une énergie potentielle, pour ensuite désactiver le dispositif.

Le troisième distributeur (33) permet de provoquer l'activation et la désactivation du pince conduit (30). Ce dernier est constitué par un étrier mobile (37) comprenant deux pointeaux internes dirigés l'un vers l'autre (39, 40), tandis qu'une butée centrale d'appui (41) est prévue au centre dudit étrier.

Le deuxième distributeur provoque le déplacement latéral de l'étrier pour pincer soit le conduit (17a) du sérum physiologique, soit le conduit (17b) du gadolinium. A cet effet l'étrier est relié mécaniquement à un boîtier de commande (41) lui-même relié hydrauliquement au deuxième distributeur (33).

Bien entendu, l'invention n'est pas limitée aux modes de réalisation décrits et représentés à titre d'exemples, mais elle comprend aussi tous les équivalents techniques ainsi que leurs combinaisons.

## Revendications

1. Dispositif d'injection destiné à mettre en oeuvre un procédé d'injection de liquide à usage médical du type qui consiste
- à utiliser une poche souple (18) contenant ledit liquide médical (19) à injecter, ladite poche étant étanche et comprenant au moins un conduit de sortie (17, 17a, 17b) destiné à être connecté à un conduit d'injection (3) tel qu'un cathéter ou une aiguille hypodermique, intraveineuse ou autre,
- à déformer la poche souple (18) par l'intermédiaire d'un liquide moteur inerte (22) contenu dans un réservoir (69), mis en pression par des moyens de mise en pression (23), de façon à transmettre cette pression au liquide médical à injecter afin de le forcer à s'écouler dans le conduit de sortie en la plaçant dans l'enceinte interne (33), d'un boîtier (20) comprenant le liquide moteur inerte (22), puis en mettant en pression ledit liquide moteur,
tandis que les moyens de mise en pression sont constitués par une pompe à piston (24) mise en mouvement par un moteur électrique (25)
**caractérisé en ce que**
- les moyens de mise en pression (23) du liquide moteur (22) sont constitués par une pompe volumétrique à pistons (24) constituée d'une succession de pistons disposés circulairement et qui sont mis en mouvement par un plateau tournant, permettant de provoquer le déplacement d'un volume constant de liquide moteur quelle que soit la vitesse du plateau, de telle sorte qu'à chaque tour du plateau correspond un volume déterminé de mise en pression du liquide moteur permettant d'obtenir un débit régulier et constant de liquide moteur afin d'assurer un débit de produit injecté lui aussi régulier et constant, par une injection rapide qui cesse dans la seconde qui suit l'arrêt de la mise en pression du liquide moteur, et qui délivre ainsi un volume et un débit constant quelle que soit la pression et ce pour une vitesse de rotation donnée, ce qui permet de transférer dans la poche motrice un volume strictement proportionnel au nombre de tour du moteur et ce quelle que soit la pression,
et **en ce que**
les moyens de mise en pression (23) comprennent un premier distributeur (26) relié à la pompe (24) ainsi qu'au réservoir (69) et à l'enceinte interne (33), ledit distributeur étant commandé par un circuit électronique pour permettre soit le déplacement du liquide du réservoir (69) vers l'enceinte (33), soit inversement le déplacement du liquide de l'enceinte (33) vers le réservoir (69).

2. Dispositif d'injection selon la revendication 1, **caractérisé en ce que** l'enceinte (33) contenant le liquide moteur est constituée par le corps du boîtier et une enceinte déformable (38) réalisée ne matériau souple et déformable élastiquement.

3. Dispositif d'injection selon la revendication 2, **caractérisé en ce que** la poche souple (18') comprend deux compartiments (180, 181) comprenant deux liquides différents, et deux conduits de sortie (17a, 17b), tandis qu'il est prévu un pince conduits (30) actionné par un bloc de commande (31) de façon à condamner l'un ou l'autre des conduits de sortie (17a, ou 17b).

4. Dispositif d'injection selon la revendication 3, **caractérisé en ce que** le pince conduits (30) est constitué par un étrier mobile (37) comprenant deux pointeaux internes dirigés l'un vers l'autre (39, 40), tandis qu'une butée centrale d'appui (41) est prévue au centre de l'étrier.

5. Dispositf d'injection selon la revendication 4, **caractérisé en ce que** le bloc de commande (31), comprend deux distributeurs, un deuxième distributeur (32), et un troisième distributeur (33), un accumulateur (34), ainsi qu'un régulateur de pression (35) et une vanne anti-retour (36).

6. Dispositif d'injection selon la revendication 5, **caractérisé en ce que** la tubulure (61) du réservoir de liquide moteur est reliée d'une part au premier distributeur (26) et d'autre part au troisième distributeur (33), et **en ce que** le deuxième distributeur est relié au premier distributeur (26) et au troisième distributeur (33), tandis que le conduit d'arrivée (60) est relié au deuxième distributeur (32), le pince conduits (30) étant relié au troisième distributeur (33).

## Claims

1. Injection device intended to implement a method of injecting liquid for medical use of the type that consists of
- using a flexible pouch (18) containing the said medical liquid (19) to be injected, the said pouch being impervious and comprising at least one outlet conduit (17, 17a, 17b) intended to be connected to an injection conduit (3) such as a catheter or hypodermic needle, intravenous or other,
- deforming the flexible pouch (18) by means of an inert driving liquid (22) contained in a reservoir (69) which is pressurised by pressurisation means (23) so as to transmit this pressure to the medical liquid to be injected in order to force it to flow in the outlet conduit by placing it in the internal chamber (33) of a housing (20) comprising the inert driving liquid (22), and then pressurising the said driving liquid,
while the pressurisation means consist of a piston pump (24) moved by an electric motor (25),
**characterised in that**
- the means (23) of pressurising the driving liquid (22) consist of a volumetric piston pump (24) consisting of a succession of pistons disposed in a circle and which are moved by a rotating plate, causing the movement of a constant volume of driving liquid whatever the speed of the plate, so that to each turn of the plate there corresponds a given pressurisation volume of the driving liquid making it possible to obtain a regular and constant flow of driving liquid in order to ensure a flow of injected product that is also regular and constant, by a rapid injection that ceases in the second following the stoppage of the pressurisation of the driving liquid, and which thus delivers a constant volume and flow rate whatever the pressure, and this for a given rotation speed, which makes it possible to transfer into the driving pouch a volume strictly proportional to the number of motor turns, and this whatever the pressure,
and **in that**
the pressurisation means (23) comprise a first control valve (26) connected to the pump (24) as well as to the reservoir (69) and to the internal chamber (33), the said control valve being controlled by an electronic circuit to allow either the movement of the liquid from the reservoir (69) to the chamber (33), or conversely the movement of the liquid from the chamber (33) to the reservoir (69).

2. Injection device according to claim 1, **characterised in that** the chamber (33) containing the driving liquid consists of the body of the housing and a deformable chamber (38) produced from flexible resiliently deformable material.

3. Injection device according to claim 2, **characterised in that** the flexible pouch (18') comprises two compartments (180, 181) comprising two different liquids, and two outlet pipes (17a, 17b), while there is provided a pipe clamp (30) actuated by a control unit (31) so as to block one or other of the outlet pipes (17a or 17b).

4. Injection device according to claim 3, **characterised in that** the pipe clamp (30) consists of a movable stirrup (37) comprising two internal needles directed towards each other (39, 40), while a central abutment stop (41) is provided at the centre of the stirrup.

5. Injection device according to claim 4, **characterised in that** the control unit (31) comprises two control valves, a second control valve (32) and a third control valve (33), an accumulator (34) as well as a pressure regulator (35) and an non-return valve (36).

6. Injection device according to claim 5, **characterised in that** the manifold (61) of the driving liquid reservoir is connected to the first control valve (26) and to the third control value (33), and **in that** the second control valve is connected to the first control value (26) and to the third control valve (33), while the inlet pipe (60) is connected to the second control valve (32), the pipe clamp (30) being connected to the third control valve (33).

## Patentansprüche

1. Injektionsvorrichtung zur Durchführung eines Verfahrens zur Injektion einer für eine medizinische Verwendung bestimmte Flüssigkeit, das darin besteht
- einen weichen Beutel (18) zu verwenden, der die zu injizierende medizinische Flüssigkeit (19) enthält, wobei der Beutel dicht ist und mindestens eine Ausgangsleitung (17, 17a, 17b) umfasst, die mit einer Injektionsleitung (3), wie zum Beispiel einem Katheter oder einer hypodermalen, intravenösen oder sonstigen Nadel, verbindbar ist,
- den weichen Beutel (18) über eine in einem Behälter (69) enthaltene inerte Antriebsflüssigkeit (22) zu verformen, welche über Druckmittel (23) derart druckbeaufschlagt ist, dass sie diesen Druck an die zu injizierende medizinische Flüssigkeit weiterleitet, damit diese in die Ausgangsleitung zwangsgeleitet wird, wobei dieser Beutel in den die inerte Antriebsflüssigkeit (22) fassenden Innenraum (33) eines Gehäuses (20) gelegt wird, und diese Antriebsflüssigkeit dann mit Druck beaufschlagt wird,
während die Druckmittel aus einer Kolbenpumpe (24) bestehen, die von einem Elektromotor (25) in Bewegung versetzt wird,
**dadurch gekennzeichnet, dass**
- die Druckmittel (23) der Antriebsflüssigkeit (22) aus einer volumetrischen Kolbenpumpe (24) bestehen, welche aus kreisförmig nacheinander angeordneten Kolben besteht, die über eine Drehplatte in Bewegung versetzt werden, wobei, unabhängig von der Geschwindigkeit der Platte, ein konstantes Volumen der Antriebsflüssigkeit verschoben werden kann, so dass jeder Umdrehung der Platte eine Druckbeaufschlagung eines bestimmten Volumens der Antriebsflüssigkeit entspricht, so dass eine regelmäßige und konstante Durchflussrate der Antriebsflüssigkeit erzielt wird, um eine ebenfalls regelmäßige und konstante Durchflussrate des injizierten Produkts zu gewährleisten, über eine schnelle Injektion, die unverzüglich nach dem Beenden der Druckbeaufschlagung der Antriebsflüssigkeit gestoppt wird, und die auf diese Weise unabhängig vom Druck und bei einer gegebenen Drehgeschwindigkeit ein konstantes Volumen und eine konstante Durchflussrate liefert, wodurch das Volumen, das in den Antriebsbeutel geleitet wird, druckunabhängig streng proportional zur Umdrehungszahl des Motors ist,
und dass
die Druckmittel (23) einen ersten mit der Pumpe (24) sowie mit dem Behälter (69) verbundenen Verteiler (26) umfassen, wobei der Verteiler von einer elektronischen Schaltung gesteuert wird, um entweder die Verschiebung der Flüssigkeit vom Behälter (69) zum Raum (33) oder umgekehrt vom Raum (33) zum Behälter (69) zu ermöglichen.

2. Injektionsvorrichtung nach Anspruch 1, **dadurch gekennzeichnet, dass** der die Antriebsflüssigkeit fassende Raum (33) aus dem Gehäusekörper und aus einer aus einem weichen und elastisch verformbaren Material bestehenden Raum (38) besteht.

3. Injektionsvorrichtung nach Anspruch 2, **dadurch gekennzeichnet, dass** der weiche Beutel (18') zwei Kammern (180, 181) mit zwei unterschiedlichen Flüssigkeiten und zwei Ausgangsleitungen (17a, 17b) umfasst, während eine von einem Steuerblock (31) betätigte Leitungsklemme (30) zum Abklemmen der einen oder der anderen Ausgangsleitung (17a bzw. 17b) vorgesehen ist.

4. Injektionsvorrichtung nach Anspruch 3, **dadurch gekennzeichnet, dass** die Leitungsklemme (30) aus einem beweglichen Bügel (37) besteht, der zwei einander zugewandte innere Stifte (39, 40) umfasst, während in der Mitte des Bügels ein mittlerer Anschlag (41) vorgesehen ist.

5. Injektionsvorrichtung nach Anspruch 4, **dadurch gekennzeichnet, dass** der Steuerblock (31) zwei Verteiler, einen zweiten Verteiler (32) und einen dritten Verteiler (33), einen Akkumulator (34) sowie einen Druckregler (35) und ein Rückschlagventil (36) umfasst.

6. Injektionsvorrichtung nach Anspruch 5, **dadurch gekennzeichnet, dass** das Rohr (61) des die Antriebsflüssigkeit fassenden Behälters einerseits mit dem ersten Verteiler (26) und andererseits mit dem dritten Verteiler (33) verbunden ist, und dass der zweite Verteiler mit dem ersten Verteiler (26) und dem dritten Verteiler (33) verbunden ist, während die Zugangsleitung (60) mit dem zweiten Verteiler (32) verbunden ist, wobei die Leitungsklemme (30) mit dem dritten Verteiler (33) verbunden ist.
